# EUROPEAN PATENT APPLICATION

(11) **EP 1 588 709 A1**
(43) Date of publication of application: **26.10.2005**
(21) Application number: 04706352.4
(22) Date of filing: 29.01.2004
(51) Int. Cl.: A61K 31/4745, C07D 471/04, A61P 3/02, A61P 43/00

(54) **VITAMIN COMPRISING PYROLOQUINOLINE QUINONE AND USE THEREOF**

(30) Priority: 30.01.2003 JP 2003021499
(71) Applicant: RIKEN, Wako-shi, Saitama 351-0198 (JP)
(72) Inventor: KASAHARA, Takaoki, Wako-shi, Saitama 351-0198 (JP); KATO, Tadafumi, Wako-shi, Saitama 351-0198 (JP)
(74) Representative: Sternagel, Fleischer, Godemeyer & Partner
(86) International application number: PCT/JP2004/000870
(87) International publication number: WO 2004/093877

(57) **Abstract**

It is an object of the present invention to clarify the biochemical role of pyrroloquinoline quinone (PQQ) in living bodies by identifying an enzyme that uses PQQ as a coenzyme in mammals and then by clarifying the oxidation-reduction reaction, with which PQQ is associated as a coenzyme in living bodies. The present invention provides a method of using pyrroloquinoline quinone as a coenzyme for 2-aminoadipate 6-semialdehyde dehydrogenase.

SEQUENCE LISTING

<110> RIKEN

<120> A vitamin pyrroloquinoline quinone and its use

<130> A31759A

<160> 12

<210> 1

<211> 3303

<212> DNA

<213> mouse

<400> 1

atgactcttc aggagttggt gcttcggact gcctctgtct atatggatag aacagctgta 60

tgttttgacg aaggcaataa ccagcctcca gtgtgctatt cctacaaggc tctgttgagc 120

gcggcttcag aattatccca ttttctgata gcacactgtg actttggagg aattcgggaa 180

attggtctct attgtcaacc tgggataaac ttaccctcct ggattttagg gattcttcag 240

gtccccgcag cgtatgcgcc cattgatcca gactccccac cgtccttatc aacttacttt 300

atgaaaaaat gtgacctaaa gtatgttctc gttgaaaaac agcagcttag taaattcaaa 360

tcttcacatg agacagttct gaactatgac acagtttccg tggaacacaa ggacctggca 420

ctcttcagac tgcattggga agatggtcgg gtgagcacag tgcttggtga cagagcagat 480

cagcacaagg tgacggatag agaagacaga gtgagtgctg agagcaggac cccagagaag 540

gagcacatgg acatgcggca tgacggttgc ttggcttatg tcctccatac ctcagggacc 600

acggggacac cgaagattgt cagagtgccc catgcatgca tactgcctaa tatccagcac 660

ttccggtcac tttttgacat cactcaagaa gacattttgt ttctggcttc tcctctgacc 720

ttcgatccat ctgttgtgga gatatttgtt tctctgtcca gtggggcctg cctgcttatc 780

gtgccaactt ctgtcaaagt gctaccatca aaattagctg acattctctt ttcccgccac 840

agagtgactg ttttacaggc aacgccaaca ttgctgagaa gatttggatc tgagctcatc 900

aagtccactg tcctgtcagc gcatacgtct ctgagagtcc tggccctcgg tggggaagcc 960

ttcccctcgc tgaccatcct caaaagctgg cgaggcaaag gcaacagaac ccagatattt 1020

aacatctatg gtatcacaga ggtatccagc tgggccactt tttacaggat cccagaggag 1080
attcttaatt ctgccgtgaa acatgaatcc cctgtgcagc tggggtcgcc actacttgga 1140
acggtgattg aagtcagaga ccaaaatggt tctccagttc tcgaaggcac tggccaagta 1200
tttttaggtg ggaagaacag agtgtgtttt cttgatgatg aaatgacagt gccccttggc 1260
accatgagag ccacaggaga ctttgtgact gtgaaagatg gagagatatt tttcttggga 1320
agaaaggaca gccagatcaa gcgccatggc aaacgtctta acattgcact tgtgcaacag 1380
gttgctgaag aacttcgcca ggtggagtcc tgcgcggtca cttggtataa tcaggaaaga 1440
ttgattctct tcatcgtatc caaagttgac ttagtaaagg actgcatctt taaagaattg 1500
cagaaacacc ttccagcaca cgccctgcct gatgacatgg tgctgatcga taccctgcca 1560
tttacatgcc atggcaaagt tgatgtttct gagttaaaca agatatattt agactacata 1620
agctcacagc ctaggaatga actccatgga aaagaggaac tttggggaaa attacagtat 1680
ttatggaagt ctattctgtg tctccctgag gatccagagg atactctgaa ggttcccgcc 1740
aactccgtct ttttagatag tggtggggat tccctcaagt ccatgcggct cctcagtgag 1800
attgagaggc tcaccgggac agccatccct ggccttctgg aagttatcct cagcagttcc 1860
ctcttagacg tttacaacca catcgttcaa gccgtgttca caccggagga caggaaagcc 1920
aacaggagtt acaccacaaa aaggaaattc agtgatgctg atccagagga agccagcggg 1980
aagcctgcac gtctggagtc tgcctggcct tcgaaccatg ccggcgagac caactccgtg 2040
atcgctctga gcagagggag tcaggttttg tctcttggcg ctgggaggct tttaactcag 2100
ttaggactct gcctgccagt ctgttctctg gatttaatcc cacagactaa cactcagata 2160
ttaaaaagcc taagtcctcc agctcctgat gaaaacttgg aaaagccccc actttttcag 2220
caggggagcc ccgtggttgg ggctatggcg atggcattgc gggagaggtg gcggtcagac 2280
acaggcaaat gcgttgatgc ctcccctctg ctggtgagag cggctgtaca ggataagccc 2340
tccacaaccg tgtatattgg ctcgcactcg cacacagtga aggctgtcga cctgtcctct 2400
ggggagacga ggtgggaaca gcttctggga gatcgaattg aatcctcagc gtgtgtgtct 2460
aaatgtggaa actttattgt agtgggctgt tataatggat tagtctatgt cctaaaaagt 2520
aatagtggag aaaaatattg gacatttact actgaagatg ctgtcaaaag ctcaccagcc 2580
gtggatccaa ccacaggact catttatgtc ggatctcatg accagcacgc atatgcttta 2640

gacatttatg aaaagaaatg tgtctggaag ctaaactgtg aaggcgctct cttttcctct 2700
ccgtgtgtga gcctgagtcc acaccatctg tactgtgcta cgctaggggg actcttactg 2760
gccctaaatc ctgcttctgg gagcacggtg tggaagcgct cctgtggaaa gccactcttc 2820
tcttccccac ggtgctacca gcaatacatc tgcatcggct gcgtggacgg cagtttgctc 2880
tgtttcacac actcagggga gcaggtctgg cgctttgctg ctggagggcc aatcttctca 2940
tccccatgcg tctcggcagc agaacaagaa atattttttg gttcccatga ctgctttatc 3000
tactgctgta gcaaggaagg tcacctccgg tggaaatttg agacgacagc cagggtgtat 3060
gcaacgccct ttgctttcag taaccacccc cgtagcgatg atgcactgct ggcagcggca 3120
tccactgacg ggaaactgtg ggtcctcgag tctcggagcg gagagctgcg aagtgtgtat 3180
gaactccccg gagaagtctt ctcctctcct gtggtctggg aatcaatgct tgttattggg 3240
tgcagaaaca attatattta ctgtctggat ttattgtgtg gtgataaaaa taaccaggtg 3300
tag 3303
<210> 2
<211> 1100
<212> PRT
<213> mouse
<400> 2
Met Thr Leu Gln Glu Leu Val Leu Arg Thr Ala Ser Val Tyr Met Asp
1               5                   10                  15
Arg Thr Ala Val Cys Phe Asp Glu Gly Asn Asn Gln Pro Pro Val Cys
            20                  25                  30
Tyr Ser Tyr Lys Ala Leu Leu Ser Ala Ala Ser Glu Leu Ser His Phe
        35                  40                  45
Leu Ile Ala His Cys Asp Phe Gly Gly Ile Arg Glu Ile Gly Leu Tyr
    50                  55                  60
Cys Gln Pro Gly Ile Asn Leu Pro Ser Trp Ile Leu Gly Ile Leu Gln
65                  70                  75                  80

Val Pro Ala Ala Tyr Ala Pro Ile Asp Pro Asp Ser Pro Pro Ser Leu
                85                  90                  95
Ser Thr Tyr Phe Met Lys Lys Cys Asp Leu Lys Tyr Val Leu Val Glu
            100                 105                 110
Lys Gln Gln Leu Ser Lys Phe Lys Ser Ser His Glu Thr Val Leu Asn
        115                 120                 125
Tyr Asp Thr Val Ser Val Glu His Lys Asp Leu Ala Leu Phe Arg Leu
    130                 135                 140
His Trp Glu Asp Gly Arg Val Ser Thr Val Leu Gly Asp Arg Ala Asp
145                 150                 155                 160
Gln His Lys Val Thr Asp Arg Glu Asp Arg Val Ser Ala Glu Ser Arg
                165                 170                 175
Thr Pro Glu Lys Glu His Met Asp Met Arg His Asp Gly Cys Leu Ala
            180                 185                 190
Tyr Val Leu His Thr Ser Gly Thr Thr Gly Thr Pro Lys Ile Val Arg
        195                 200                 205
Val Pro His Ala Cys Ile Leu Pro Asn Ile Gln His Phe Arg Ser Leu
    210                 215                 220
Phe Asp Ile Thr Gln Glu Asp Ile Leu Phe Leu Ala Ser Pro Leu Thr
225                 230                 235                 240
Phe Asp Pro Ser Val Val Glu Ile Phe Val Ser Leu Ser Ser Gly Ala
                245                 250                 255
Cys Leu Leu Ile Val Pro Thr Ser Val Lys Val Leu Pro Ser Lys Leu
            260                 265                 270
Ala Asp Ile Leu Phe Ser Arg His Arg Val Thr Val Leu Gln Ala Thr
        275                 280                 285
Pro Thr Leu Leu Arg Arg Phe Gly Ser Glu Leu Ile Lys Ser Thr Val

    290                 295                 300
Leu Ser Ala His Thr Ser Leu Arg Val Leu Ala Leu Gly Gly Glu Ala
305                 310                 315                 320
Phe Pro Ser Leu Thr Ile Leu Lys Ser Trp Arg Gly Lys Gly Asn Arg
                325                 330                 335
Thr Gln Ile Phe Asn Ile Tyr Gly Ile Thr Glu Val Ser Ser Trp Ala
            340                 345                 350
Thr Phe Tyr Arg Ile Pro Glu Glu Ile Leu Asn Ser Ala Val Lys His
        355                 360                 365
Glu Ser Pro Val Gln Leu Gly Ser Pro Leu Leu Gly Thr Val Ile Glu
    370                 375                 380
Val Arg Asp Gln Asn Gly Ser Pro Val Leu Glu Gly Thr Gly Gln Val
385                 390                 395                 400
Phe Leu Gly Gly Lys Asn Arg Val Cys Phe Leu Asp Asp Glu Met Thr
                405                 410                 415
Val Pro Leu Gly Thr Met Arg Ala Thr Gly Asp Phe Val Thr Val Lys
            420                 425                 430
Asp Gly Glu Ile Phe Phe Leu Gly Arg Lys Asp Ser Gln Ile Lys Arg
        435                 440                 445
His Gly Lys Arg Leu Asn Ile Ala Leu Val Gln Gln Val Ala Glu Glu
    450                 455                 460
Leu Arg Gln Val Glu Ser Cys Ala Val Thr Trp Tyr Asn Gln Glu Arg
465                 470                 475                 480
Leu Ile Leu Phe Ile Val Ser Lys Val Asp Leu Val Lys Asp Cys Ile
                485                 490                 495
Phe Lys Glu Leu Gln Lys His Leu Pro Ala His Ala Leu Pro Asp Asp
            500                 505                 510

Met Val Leu Ile Asp Thr Leu Pro Phe Thr Cys His Gly Lys Val Asp
        515                 520                 525
Val Ser Glu Leu Asn Lys Ile Tyr Leu Asp Tyr Ile Ser Ser Gln Pro
    530                 535                 540
Arg Asn Glu Leu His Gly Lys Glu Glu Leu Trp Gly Lys Leu Gln Tyr
545                 550                 555                 560
Leu Trp Lys Ser Ile Leu Cys Leu Pro Glu Asp Pro Glu Asp Thr Leu
                565                 570                 575
Lys Val Pro Ala Asn Ser Val Phe Leu Asp Ser Gly Gly Asp Ser Leu
            580                 585                 590
Lys Ser Met Arg Leu Leu Ser Glu Ile Glu Arg Leu Thr Gly Thr Ala
        595                 600                 605
Ile Pro Gly Leu Leu Glu Val Ile Leu Ser Ser Ser Leu Leu Asp Val
610 625 620
Tyr Asn His Ile Val Gln Ala Val Phe Thr Pro Glu Asp Arg Lys Ala
625                 630                 635                 640
Asn Arg Ser Tyr Thr Thr Lys Arg Lys Phe Ser Asp Ala Asp Pro Glu
                645                 650                 655
Glu Ala Ser Gly Lys Pro Ala Arg Leu Glu Ser Ala Trp Pro Ser Asn
            660                 665                 670
His Ala Gly Glu Thr Asn Ser Val Ile Ala Leu Ser Arg Gly Ser Gln
        675                 680                 685
Val Leu Ser Leu Gly Ala Gly Arg Leu Leu Thr Gln Leu Gly Leu Cys
    690                 695                 700
Leu Pro Val Cys Ser Leu Asp Leu Ile Pro Gln Thr Asn Thr Gln Ile
705                 710                 715                 720
Leu Lys Ser Leu Ser Pro Pro Ala Pro Asp Glu Asn Leu Glu Lys Pro

                725                 730                 735
Pro Leu Phe Gln Gln Gly Ser Pro Val Val Gly Ala Met Ala Met Ala
            740                 745                 750
Leu Arg Glu Arg Trp Arg Ser Asp Thr Gly Lys Cys Val Asp Ala Ser
        755                 760                 765
Pro Leu Leu Val Arg Ala Ala Val Gln Asp Lys Pro Ser Thr Thr Val
    770                 775                 780
Tyr Ile Gly Ser His Ser His Thr Val Lys Ala Val Asp Leu Ser Ser
785                 790                 795                 800
Gly Glu Thr Arg Trp Glu Gln Leu Leu Gly Asp Arg Ile Glu Ser Ser
                805                 810                 815
Ala Cys Val Ser Lys Cys Gly Asn Phe Ile Val Val Gly Cys Tyr Asn
            820                 825                 830
Gly Leu Val Tyr Val Leu Lys Ser Asn Ser Gly Glu Lys Tyr Trp Thr
        835                 840                 845
Phe Thr Thr Glu Asp Ala Val Lys Ser Ser Pro Ala Val Asp Pro Thr
    850                 855                 860
Thr Gly Leu Ile Tyr Val Gly Ser His Asp Gln His Ala Tyr Ala Leu
865                 870                 875                 880
Asp Ile Tyr Glu Lys Lys Cys Val Trp Lys Leu Asn Cys Glu Gly Ala
                885                 890                 895
Leu Phe Ser Ser Pro Cys Val Ser Leu Ser Pro His His Leu Tyr Cys
            900                 905                 910
Ala Thr Leu Gly Gly Leu Leu Leu Ala Leu Asn Pro Ala Ser Gly Ser
        915                 920                 925
Thr Val Trp Lys Arg Ser Cys Gly Lys Pro Leu Phe Ser Ser Pro Arg
    930                 935                 940

Cys Tyr Gln Gln Tyr Ile Cys Ile Gly Cys Val Asp Gly Ser Leu Leu
945                 950                 955                 960
Cys Phe Thr His Ser Gly Glu Gln Val Trp Arg Phe Ala Ala Gly Gly
                965                 970                 975
Pro Ile Phe Ser Ser Pro Cys Val Ser Ala Ala Glu Gln Glu Ile Phe
            980                 985                 990
Phe Gly Ser His Asp Cys Phe Ile Tyr Cys Cys Ser Lys Glu Gly His
        995                 1000                1005
Leu Arg Trp Lys Phe Glu Thr Thr Ala Arg Val Tyr Ala Thr Pro Phe
    1010                1015                1020
Ala Phe Ser Asn His Pro Arg Ser Asp Asp Ala Leu Leu Ala Ala Ala
1025                1030                1035                1040
Ser Thr Asp Gly Lys Leu Trp Val Leu Glu Ser Arg Ser Gly Glu Leu
                1045                1050                1055
Arg Ser Val Tyr Glu Leu Pro Gly Glu Val Phe Ser Ser Pro Val Val
            1060                1065                1070
Trp Glu Ser Met Leu Val Ile Gly Cys Arg Asn Asn Tyr Ile Tyr Cys
        1075                1080                1085
Leu Asp Leu Leu Cys Gly Asp Lys Asn Asn Gln Val
    1090                1095                1100
<210> 3
<211> 23
<212> DNA
<213> Artificial Sequence
<220>
<223> Description of Artificial Sequence: Synthetic DNA
<400> 3

aactcctgag atttctcagc cac 23
<210> 4
<211> 23
<212> DNA
<213> Artificial Sequence
<220>
<223> Description of Artificial Sequence: Synthetic DNA
<400> 4
acatttccaa ccctctcact gac 23
<210> 5
<211> 19
<212> DNA
<213> Artificial Sequence
<220>
<223> Description of Artificial Sequence: Synthetic DNA
<400> 5
tgcaccacca actgcttag 19
<210> 6
<211> 19
<212> DNA
<213> Artificial Sequence
<220>
<223> Description of Artificial Sequence: Synthetic DNA
<400> 6
ggatgcaggg atgatgttc 19
<210> 7
<211> 22

<212> DNA

<213> Artificial Sequence

<220>

<223> Description of Artificial Sequence: Synthetic DNA

<400> 7

cgcagaccag caaattattc ac                                22

<210> 8

<211> 20

<212> DNA

<213> Artificial Sequence

<220>

<223> Description of Artificial Sequence: Synthetic DNA

<400> 8

attccgtagc ctcgattgga                                   20

<210> 9

<211> 20

<212> DNA

<213> Artificial Sequence

<220>

<223> Description of Artificial Sequence: Synthetic DNA

<400> 9

ctggtgagag cggctgtaca                                   20

<210> 10

<211> 20

<212> DNA

<213> Artificial Sequence

<220>

<223> Description of Artificial Sequence: Synthetic DNA

<400> 10

ggacaggtcg acagccttca                                    20

<210> 11

<211> 22

<212> DNA

<213> Artificial Sequence

<220>

<223> Description of Artificial Sequence: Synthetic DNA

<400> 11

tgatgaatgg actcagctgg at                                 22

<210> 12

<211> 24

<212> DNA

<213> Artificial Sequence

<220>

<223> Description of Artificial Sequence: Synthetic DNA

<400> 12

cctgaccaat gtccatgttt aatg                               24

## Description

### TECHNICAL FIELD

The present invention relates to pyrroloquinoline quinone acting as a coenzyme for 2-aminoadipate 6-semialdehyde dehydrogenase and a use thereof.

### BACKGROUND ART

When amino acids or sugars are decomposed in bodies, various organic substances function as cofactors of metabolizing enzymes. Among these cofactors, nicotinamide compounds (NAD and NADP) and flavin compounds (FAD and FMN) have been known as oxidation-reduction coenzymes supporting an oxidation-reduction reaction. In 1979, a novel oxidoreductase that supports methanol dehydrogenase of methanol-assimilating bacteria was discovered, and it was named as pyrroloquinoline quinone (which is abbreviated as PQQ at time, and the chemical structure of which is shown below).

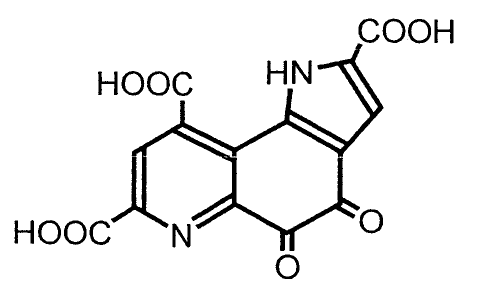

Thereafter, it has become clear that PQQ functions as a coenzyme for dehydrogenases of various prokaryotes such as *Escherichiα coli.* Unexpectedly, it has been clarified that PQQ is contained not only in prokaryotes but also in plants (vegetables) and animals (meats). As a result of experiments in which mice were fed with feedstuff that contained no PQQ, it was found that various types of abnormalities were developed due to the absence of PQQ. PQQ-deficient mice have fragility in the skin, osteolathyrism-like skeletal abnormality, and a decrease in immune response. Although such PQQ-deficient mice were able to become pregnant, a majority of mothers ate their newborn pups after the birth. Moreover, the growth of surviving pups mice was unfavorable. These abnormalities were recovered by addition of a small amount of PQQ to feedstuff (800 ng or more per gram of feedstuff). PQQ can be synthesized only by certain types of bacteria. The amount of PQQ synthesized by enteric bacteria is below a measurable level. Thus, it has been suggested that for mammals PQQ is an important nutrient, which should be incepted from the outside. However, the biochemical role of PQQ in living bodies has remained unknown (Killgore, J., Smidt, C., Duich, L., Romero-Chapman, N., Tinker, D., Reiser, K., Melko, M., Hyde, D., and Rucker, R.B. (1989) Nutritional importance of pyrroloquinoline quinone. Science 245, 850-852; Smidt, C.R., Bean-Knudsen, D., Kirsch, D.G., and Rucker, R.B. (1991) Does the intestinal microflora synthesize pyrroloquinoline quinone? Biofactors 3, 53-59; Steinberg, F.M., Gershwin, M.E., and Rucker, R.B. (1994) Dietary pyrroloquinoline quinone: growth and immune response in BALB/c mice. J. Nutr. 124, 744-753; and Stites, T.E., Mitchell, A.E., and Rucker, R.B. (2000) Physiological importance of quinoenzymes and the o-quinone family of cofactors. J. Nutr. 130, 719-727).

### DISCLOSURE OF THE INVENTION

It is an object of the present invention to clarify the biochemical role of pyrroloquinoline quinone (PQQ) in living bodies by identifying an enzyme that uses PQQ as a coenzyme in mammals and then by clarifying the oxidation-reduction reaction, with which PQQ is associated as a coenzyme in living bodies. It is another object of the present invention to provide a novel vitamin preparation by focusing attention on the biochemical role of PQQ in living bodies.

As a result of intensive studies directed towards achieving the aforementioned objects, the present inventors have succeeded in identifying 2-aminoadipate 6-semialdehyde dehydrogenase as an enzyme that uses pyrroloquinoline quinone (PQQ) as a coenzyme in mammals. In addition, as a result of the identification of this enzyme, it was confirmed that PQQ is an important nutrient as a novel type of vitamin in animals including humans.

That is to say, the present invention provides a method of using pyrroloquinoline quinone as a coenzyme for 2-aminoadipate 6-semialdehyde dehydrogenase.

In another aspect, the present invention provides a method for converting 2-aminoadipate 6-semialdehyde into 2-aminoadipic acid, which comprises allowing 2-aminoadipate 6-semialdehyde dehydrogenase to act on 2-aminoadipate 6-semialdehyde in the presence of pyrroloquinoline quinone.

In further another aspect, the present invention provides a vitamin preparation which comprises pyrroloquinoline quinone as a coenzyme for 2-aminoadipate 6-semialdehyde dehydrogenase.

### BRIEF DESCRIPTION OF THE DRAWINGS

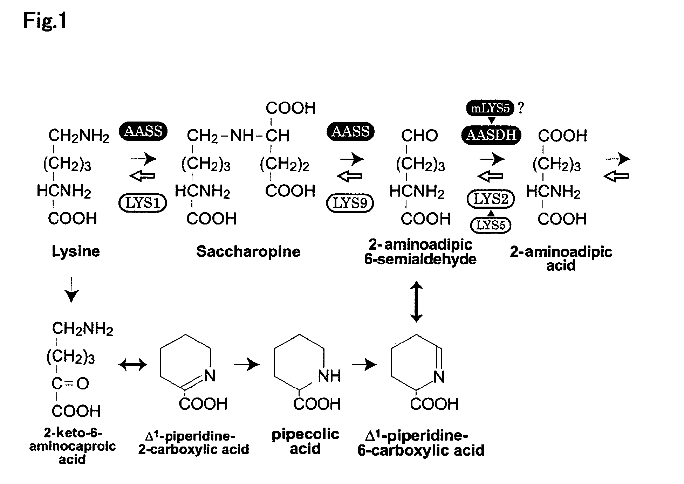

Figure 1 shows a lysine metabolic pathway in a mammal and yeast.

Two pathways, that are, a saccharopine route (upper) and a pipecolate route (lower), exist in a lysine degradative pathway (filled arrows) in the mammal. The lysine degradative pathway in the yeast *(S. cerevisiae)* is indicated with open arrows. Double-headed arrows represent autonomic reactions. For example, 2-aminoadipate 6-semialdehyde (AAS) and Δ¹-piperideine-6-carboxylate (P6C) are in an equilibrium state in living bodies. LYS2 is post-translationally 4'-phosphopantetheinylated catalyzed by LYS5. AASS represents AAS synthetase; AASDH represents AAS dehydrogenase; and mLYS5 represents a mammalian homologue of yeast LYS5.

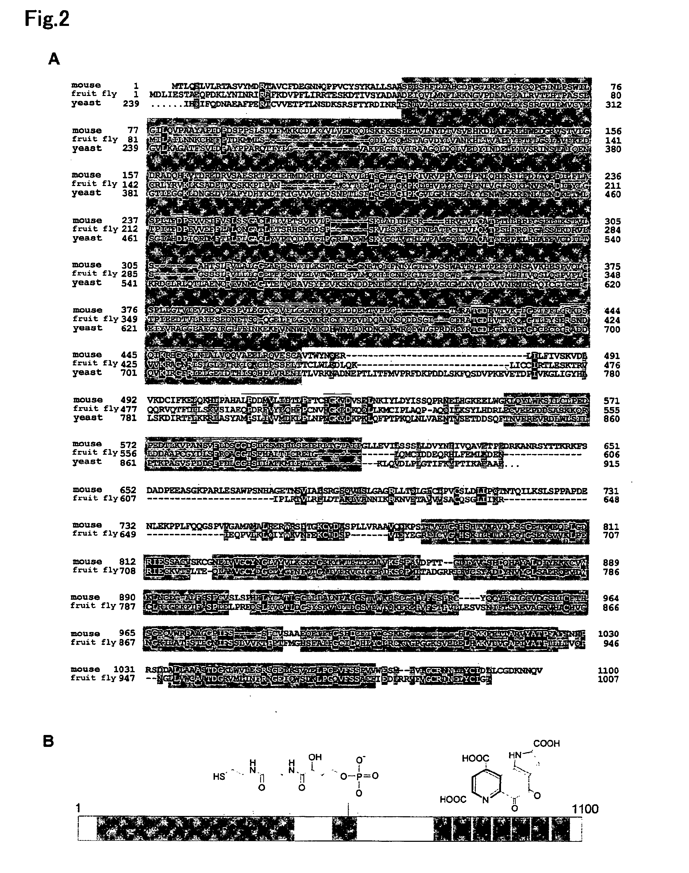

Figure 2 shows the structure of mouse U26.

An adenylation domain (Pfam00501), a thiolation domain (Pfam00550), and a PQQ-binding motif (Pfam01011) are indicated with pink, green, and light blue, respectively.
(A) represents an alignment of the putative amino acid sequences of portions (ILe²³⁹-Glu⁹¹⁵) from mouse U26 (mouse), fruit fly U26 (fly), and yeast LES2 (yeast). Black background (reverse characters) represents amino acid residues that are identical among the aligned proteins. Grey background represents amino acid residues that are not identical among the aligned proteins but the properties of which are similar to one another. In addition, the horizontal lines represent regions corresponding to core sequences that are well conserved among a group of non-ribosome peptide synthetases of bacteria.
(B) represents the structure of a functional domain of mouse U26 and the molecular structure of a cofactor. A serine residue (Ser⁵⁹²) in the thiolation domain is converted into 4'-phosphopantetheine as a result of post-translational modification. In addition, PQQ can be non-covalently bounded by 7 consecutive PQQ-binding motifs.

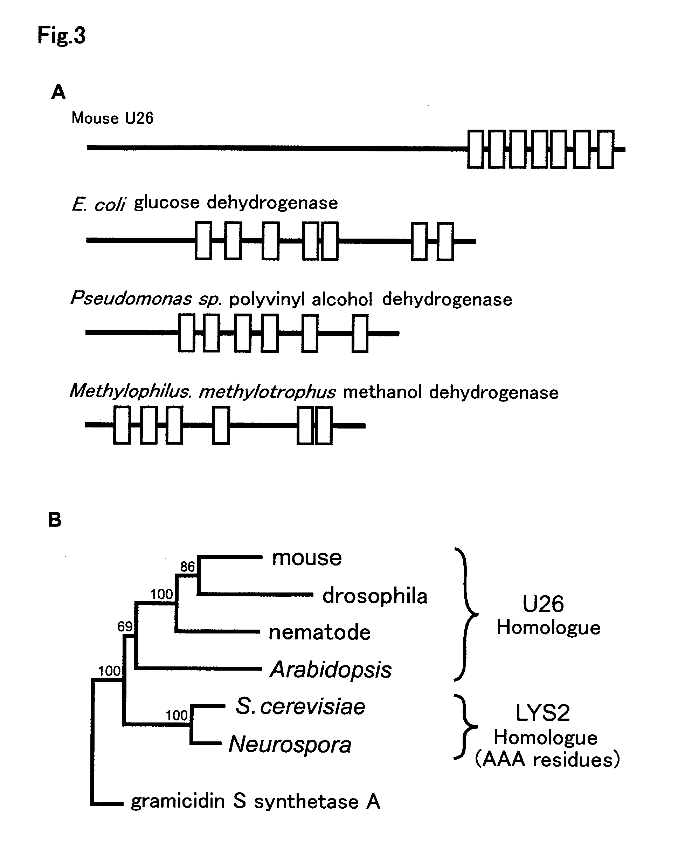

Figure 3 shows a comparison between the mouse U26 protein and other types of related proteins.
(A) represents the domain structure of PQQ-binding motifs. The horizontal line represents the overall length of a protein, and the square represents a PQQ-binding motif. Accession numbers to public database are as follows. *E. coli* glucose dehydrogenase: P15877; *Pseudomonas sp.* polyvinyl alcohol dehydrogenase: BAA94193; and *M. methylotrophus* methanol dehydrogenase: P38539.
(B) represents a phylogenetic tree of U26 and LYS2 homologue. The tree is constructed by the neighbor-joining method on the basis of the amino acid sequence of an A/T domain (corresponding to amino acid residues from positions 34 to 626 of mouse U26). Gramicidin S synthetase A of *Bacillus brevis* was used as an outgroup. The length of the horizontal line represents an evolutionary distance, and the numerical value of each node represents a bootstrap probability (%) obtained based on 1,000 times of trials. Accession numbers to public database are as follows. *Neurospora* LYS2 homologue: CAB97293; and *B. brevis* gramicidin S synthetase A: P14687.

Figure 4 shows a tissue distribution of the expression of mU26 mRNA.

A blot was purchased from Clontech, and ³²P-labeled mU26 cDNA was used as a probe. 2 µg of poly(A)⁺RNA was electrophoresed in each lane.

Figure 5 shows the effects of high lysine feedstuff on the mRNA level of a lysine metabolic enzyme.
(A and B) represent the level of lysine (A) in blood and the level of 2-aminoadipic acid (B) in blood, in mice which were fed with high lysine feedstuff (filled circle) and with ordinary feedstuff (open circle). The amount of lysine is indicated with a percentage to the total amount of amino acids in blood. 2-aminoadipic acid is indicated after being normalized with glycine in blood. Values in the figure represent mean values ± standard deviations.
(C) represents the mRNA level of a lysine metabolic enzyme. The heart and liver were collected on the 20^{th} day after initiation of the administration of high lysine feedstuff. Thereafter, the mRNA of each of mAASS, mLys5, and mGAPDH was quantified by quantitative RT-PCR. Each mRNA level was obtained as a relative value to mGAPDH, and the mean value of a control mouse was defined as 1.

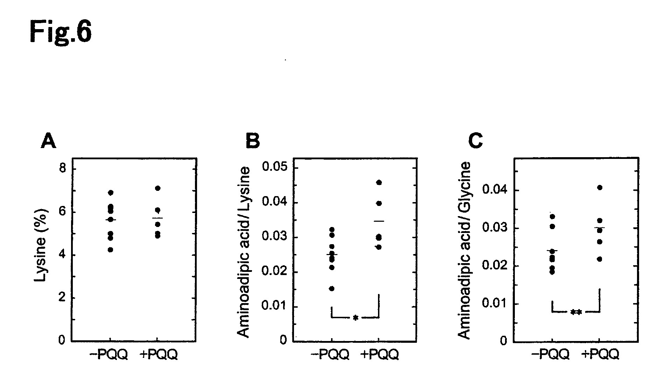

Figure 6 shows the level of lysine in blood and the level of 2-aminoadipic acid in blood in PQQ-deficient mice.

The mice were fed with feedstuff, to which PQQ had not been added (-PQQ) or had been added (+PQQ) and which had chemically been limited, for 20 days. Thereafter, the peripheral blood was collected therefrom, and amino acids contained in the blood were quantified using an amino acid analyzer. The amount of lysine (A) was indicated with a percentage to the total amount of amino acids in blood. The amount of 2-aminoadipic acid was normalized with lysine (B) or glycine (C). The horizontal line represents the mean value of mice in each group. The amount of lysine was not significantly different between PQQ-deficient mice and PQQ-added mice (t = 0.16, d. f. = 11). However, the amount of 2-aminoadipic acid in the blood of PQQ-deficient mice was reduced more significantly than that in the blood of PQQ-added mice (*: p = 0.01; **: p = 0.05).

### BEST MODE FOR CARRYING OUT THE INVENTION

The embodiments of the present invention will be described in detail below.

With regard to lysine metabolism in higher eukaryotes, an enzyme (AAS dehydrogenase; AASDH) that catalyzes a reaction of oxidizing 2-aminoadipate 6-semialdehyde (AAS) to convert it into 2-aminoadipic acid (AAA) has not yet been identified to date. The present inventors have isolated a novel gene encoding this enzyme (AAS dehydrogenase; AASDH) from a mouse. Moreover, the present inventors have found a motif binding with PQQ in the gene product. Thus, it was shown that this is a novel enzyme that utilizes PQQ in eukaryotes. It has been suggested so far that PQQ is an important nutrient for mammals. However, since the biochemical role of PQQ in living bodies has remained unknown, PQQ has not generally been considered as vitamin. The present inventors have demonstrated that the AAA concentration in blood is significantly low in PQQ-deficient mice. This fact shows that PQQ is essential for the activity of AASDH, and thus, the biochemical role of PQQ (namely, an oxidization-reduction coenzyme essential for lysine metabolism), which had remained unknown to date, has been clarified. From these results, it can be said that PQQ is ready for being considered as a novel vitamin.

As stated above, according to the present invention, 2-aminoadipate 6-semialdehyde dehydrogenase has been identified for the first time as an enzyme that utilizes pyrroloquinoline quinone as a coenzyme. The present invention includes all methods of using pyrroloquinoline quinone as a coenzyme for 2-aminoadipate 6-semialdehyde dehydrogenase. A specific example of the aforementioned methods of using pyrroloquinoline quinone may be a method for converting 2-aminoadipate 6-semialdehyde into 2-aminoadipic acid, which comprises allowing 2-aminoadipate 6-semialdehyde dehydrogenase to act on 2-aminoadipate 6-semialdehyde in the presence of pyrroloquinoline quinone. Such a reaction includes both the case of performing the reaction *in vivo,* and the case of performing the *reaction in vitro* (that is, including both the case of performing the reaction in cells, and the case of performing it in a test tube).

2-aminoadipate 6-semialdehyde is a known substance. This substance is described, for example, in Aspen A.J. et al., The preparation and some properties of α-aminoadipic-δ-semialdehyde (Δ¹-piperideine-6-carboxylic acid), Biochemistry 1, 600-605 (1962); Sacksteder K. A. et al., Identification of the α-aminoadipic semialdehyde synthase gene, which is defective in familial hyperlysinemia, Am. J. Hum. Genet. 66, 1736-1743 (2000); etc. In addition, pyrroloquinoline quinone is also a known substance, and is available from Sigma-Aldrich Japan K. K., Wako Pure Chemical Industries, Ltd., or other companies.

The nucleotide sequence of 2-aminoadipate 6-semialdehyde dehydrogenase and the amino acid sequence thereof have been registered in public database. These sequences are shown in SEQ ID NOS: 1 and 2 in the sequence listing of the present specification, respectively.

When the isolated and purified 2-aminioadipate 6-semialdehyde dehydrogenase is required, that is, when the aforementioned conversion reaction of the present invention is carried out in a test tube for example, a gene encoding the above enzyme is cloned, the cloned gene is incorporated into an expression vector, and the vector is then introduced into a host, so as to transform it. Thereafter, the obtained transformant is cultured, and 2-aminoadipate 6-semialdehyde dehydrogenase is recovered, isolated and purified from the obtained culture according to common methods. The production of a recombinant protein according to the aforementioned gene recombination technique can be carried out by methods that have been known to persons skilled in the art. On the other hand, when the aforementioned conversion reaction of the present invention is carried out in living bodies or in cells, such a conversion reaction may be carried out using 2-aminoadipate 6-semialdehyde dehydrogenase that has originally existed in such living bodies or cells.

As stated above, based on the findings of the present invention, PQQ has been recognized as a novel vitamin for the first time. That is to say, the present invention provides a vitamin preparation which comprises pyrroloquinoline quinone as a coenzyme for 2-aminoadipate 6-semialdehyde dehydrogenase.

The term "vitamin" means a group of substances essential for normal metabolism, growth and development, or the control of cell functions, which function together with enzymes, coenzymes, or other substances. In addition, vitamin is a trace amount of nutrient, and the amount required thereof cannot be fully synthesized *in vivo.* Thus, it must be taken from food or the like.

The dosage form of the vitamin preparation of the present invention is not particularly limited. An appropriate dosage form can be selected from dosage forms for oral administration or parenteral administration. Examples of a dosage form suitable for oral administration may include a tablet, a capsule, a powder, a granule, a parvule, a syrup, a solution, an emulsion, a suspension, and a chewable tablet. Examples of a dosage form suitable for parenteral administration may include an injection (for example, a subcutaneous injection, an intramuscular injection, an intravenous injection, etc.), a drop, and a medicine for external use (for example, percutaneous absorption agents such as an ointment, an adhesive preparation, or tapes). However, examples are not limited thereto. Preferred dosage forms may include those suitable for drop administration (central venous hyperalimentation), percutaneous administration, and oral administration.

Liquid preparations suitable for oral administration, such as a solution, an emulsion, or a syrup, can be produced using water, sugars such as sucrose, sorbit or fructose, glycols such as polyethylene glycol or propylene glycol, oils such as sesame oil, olive oil or soybean oil, antiseptics such as p-hydroxybenzoic acid esters, or flavors such as strawberry flavor or peppermint flavor. Moreover, solid preparations such as a capsule, a tablet, a powder, or a granule can be produced by using excipients such as lactose, glucose, sucrose, or mannite, disintegrators such as starch or sodium alginate, lubricants such as magnesium stearate or talc, binders such as polyvinyl alcohol, hydroxypropylcellulose, or gelatin, surfactants such as fatty acid ester, plasticizers such as glycerin, and so on.

Injections, drops or preparations for external use, which are suitable for parenteral administration, preferably comprise, as an active ingredient, PQQ that is dissolved or suspended in a sterilized water-type medium. In the case of an injection for example, a solution can be prepared by using an aqueous medium that is a saline solution, a glucose solution, or a mixture consisting of a saline solution and a glucose solution. One or more supplementary components selected from the group consisting of glycols, oils, flavors, antiseptics, excipients, disintegrators, lubricants, binders, surfactants, and plasticizers may be added to preparations used for parenteral administration.

Moreover, known vitamins that are used for various types of pharmaceuticals, quasi drugs, or food products may also be added to the vitamin preparation of the present invention. Specifically, the following components may be added to the vitamin preparation of the present invention: retinol acetate, retinol palmitate, vitamin A oil, cod liver oil, extra-strong cod liver oil, ergocalciferol, cholecalciferol, d-α-tocopherol acetate, dl-α-tocopherol acetate, d-α-tocopherol, dl-α-tocopherol, flavin adenin dinucleotide sodium, riboflavin, riboflavin sodium phosphate, riboflavin tetrabutyrate, pyridoxine hydrochloride, pyridoxal phosphate, hydroxocobalamin hydrochloride, hydroxocobalamin acetate, cyanocobalamin, hydroxocobalamin, nicotinic acid, nicotinic acid amide, panthenol, calcium pantothenate, sodium pantothenate, biotin, a mixture of potassium aspartate and magnesium in equal proportions, inositol hexanicotinate, ursodesoxycholic acid, L-cysteine hydrochloride, L-cysteine, orotic acid, γ-oryzanol, calcium glycerophosphate, calcium gluconate, precipitated calcium carbonate, calcium lactate, anhydrous dibasic calcium phosphate, dibasic calcium phosphate, glucuronolactone, glucuronamide, sodium chondroitin sulfate, etc. Persons skilled in the art may add one or more selected from the aforementioned components to the vitamin preparation of the present invention at an appropriate mixing ratio.

The ratio of pyrroloquinoline quinone (PQQ) contained in the vitamin preparation of the present invention is not particularly limited. It is generally approximately between 0.0001% and 10% by weight, and preferably approximately between 0.0002% and 0.5% by weight. For example, the amount of pyrroloquinoline quinone (PQQ) contained in a single vitamin preparation is within a range generally approximately between 0.001 and 10 mg, and preferably approximately between 0.002 and 5 mg.

The dosage of the vitamin preparation of the present invention and the number of administrations thereof can appropriately be determined depending on various factors such as the age, body weight, and symptoms of a patient. The dosage of pyrroloquinoline quinone (PQQ) as an active ingredient is generally approximately between 5 and 1,000 µg/kg of body weight, and preferably approximately between 15 and 500 µg/kg of body weight, per day. The vitamin preparation of the present invention can be administered to humans and other types of any given animals (for example, mammals, birds, etc.).

Pyrroloquinoline quinone (PQQ) used in the present invention is not only singly used as a vitamin preparation, but also it can be mixed into drinkable preparations such as nutritional drinks, or into health food as a food additive. Specific examples of a product comprising pyrroloquinoline quinone (PQQ) may include health foods and supplementary foods including beverages, which are generally called soft drink, drinkable preparation, health food, specified health food, functional food, function activating food, nutritional supplementary food, supplement, feed, feed additive, etc. Specific examples of the aforementioned foods and beverages may include all types of foods and beverages including: confectioneries such as chewing gum, chocolate, candy, confection, jelly, cookie, biscuit, or yogurt; frozen desserts such as ice cream or ice lolly; beverages such as tea, soft drink (including juice, coffee, cocoa, etc.), nutritional drink, or drink used in beauty treatment; bread; ham; soup; jam; spaghetti; and frozen foods. Moreover, pyrroloquinoline quinone (PQQ) can also be added to condiments or food additives. Furthermore, PQQ can be used for skin care products and hair care products such as cosmetics, shampoos, or hair growth agents. Still further, it can also be used for products for maintaining oral hygiene, such as toothpaste or mouthwash.

By administering the vitamin preparation of the present invention, the biochemical functions of pyrroloquinoline quinone (PQQ) as a coenzyme for 2-aminoadipate 6-semialdehyde dehydrogenase are exhibited, and various symptoms caused by the absence of PQQ can be thereby prevented, alleviated, or treated. Examples of such symptoms caused by the absence of PQQ may include fragility in the skin, osteolathyrism-like skeletal abnormality, and a decrease in immune response.

The present invention will be more specifically described in the following examples. However, these examples are not intended to limit the scope of the present invention.

### EXAMPLES

### Example 1: Cloning of mouse U26 gene and evaluation of domain structure

LYS2 that is 2-aminoadipic acid (AAA) reductase of yeast *(S. cerevisiae)* has an adenylation (A) domain and a thiolation (T) domain. These domains are structures found also in non-ribosome peptide synthetase (NRPS) of bacteria or molds (Morris, M. E. et al. (1991) Gene 98, 141-145; Ehmann, D. E. et al. (1999) Biochemistry 38, 6171-6177; and Konz, D. et al. (1999) Chem. Biol. 6, R39-R48, as a review of NRPS).

First, a gene product that has both the A domain and the T domain was searched through the genomic sequence database of fruit fly *(Drosophila melanogaster).* According to the TBlast search method based on sequences in which the A/T domains are conserved, EBONY (Accession No. CAA11962 to public database) and U26 (AAF52679) were found. Both these gene products had a single A domain and a single T domain. It has been considered that EBONY is β-alanyl dopamine synthetase (Hovemann, B.T. et al. (1998) Gene 221, 1-9), but the functions of U26 have remained unknown. It is important that several PQQ-binding motifs exist in the C-terminal region of U26. PQQ is a molecule, which is used as a coenzyme that causes oxidization (receiving electrons) for dehydrogenase of bacteria (Goodwin, P.M. et al. (1998) Adv. Microb. Physiol. 40, 1-80). From the fact that U26 has A/T domains and PQQ-binding motifs, it was assumed that U26 is aminoadipate 6-semialdehyde dehydrogenase (AASDH) of *Drosophila melanogaster.* However, since there was only a little information regarding lysine metabolism in insects, mammalian homologues of *Drosophila melanogaster* U26 (dU26) were studied. When human genomic sequence was searched by the TBlastN method, a sequence that is homologous with dU26 was found in the 4^{th} chromosome q12. Based on this sequence, a mouse homologue was isolated (Figure 2A). The nucleotide sequence and amino acid sequence of mouse U26 (m26) are shown in SEQ ID NOS: 1 and 2 in the sequence listing. The following synthetic DNA primers were used for reverse transcription (RT)-PCR for obtaining cDNA containing the full-length coding region.

Fw: 5'-AACTCCTGAGATTTCTCAGCCAC-3' (SEQ ID NO: 3)

Rv: 5'-ACATTTCCAACCCTCTCACTGAC-3' (SEQ ID NO: 4)

The cDNA of mouse U26 (mU26) encodes a protein of 1,100 amino acid residues. When the mouse U26 is compared with dU26, 23% of their amino acid residues were identical, and 43% of their amino acid residues were similar. mU26 also has a single A domain and a single T domain on the N-terminal side thereof, and has consecutive PQQ-binding sequences on the C-terminal side thereof (Figure 2). Using ClustalW program, the A/T domains of mU26, dU26, and yeast LYS2 were aligned and compared (Figure 2A). Several extremely well conserved portions were detected, and almost all these portions corresponded to core sequences that are essential for recognizing substrates (generally, amino acids) in NRPS (Konz, D. et al. (1999) Chem. Biol. 6, R39-R48). This means that U26 binds to substrates using the A/T domains, as in the case of NRPS or LYS2. In addition, 7-repetitive PQQ-binding motifs exist in the C-terminal region of mU26 (Figures 2, 3A). It has been known that PQQ-dependent dehydrogenase of bacteria also has a structure consisting of 6- or 7-repetitive PQQ-binding motifs (Mathews, F. S. (1995) Methods Enzymol. 258, 191-216). The domain structure of mU26 corresponds to the above structure. These results (Figure 3A) support the assumption that mU26 is dehydrogenase utilizing PQQ as an oxidization-reduction coenzyme.

### Example 2: AAS-AAA oxidoreductase family is present in phylogenetic analysis

As a result of the TBlastN search based on the sequence of mU26, it was found that nematode *(Caenorhabditis elegans)* and Arabidopsis *(Arabidopsis thaliana)* also have U26 homologues (CAB04944 and BAA97455, respectively). Both homologues had been registered in database as hypothetical proteins (hypothetical proteins predicted from genomic sequences). As with mU26 and dU26, these U26 homologues have a single A domain and a single T domain on the N-terminal side thereof, and have PQQ-binding motifs on the C-terminal side thereof (data is not shown). The fact that a wide range of higher eukaryotes have a U26 homologue matches with the fact that animals or higher plants have a lysine degradative pathway via 2-aminoadipate 6-semialdehyde (AAS).

Thus, based on theory of molecular evolution, such a U26 homologue and AAA reductase (yeast and *Neurospora crassa)* were analyzed. Based on the amino acid sequences of the A/T domains that are commonly observed in these proteins, molecular cladograms were produced. Gramicidin S synthetase A of *Bacillus brevis* (Stachelhaus, T. et al. (1995) J. Biol. Chem. 270, 6163-6169), which is a well-analyzed NRPS, was used as an outgroup (Figure 3B). U26 homologues of mouse, *Drosophila melanogaster, Caenorhabditis elegans,* and *Arabidopsis thaliana* were classified into a single cluster. AAA reductase formed another cluster. This pattern of dendrogram of being divided into branches corresponds to the pattern of phylogeny of eukaryotes. This shows that the U26 homologue and the AAA reductase have a common ancestral gene. This analysis suggested that a group of these proteins including the U26 homologue forms a novel enzyme family that catalyzes an oxidization-reduction reaction AAS ←→ AAA.

### Example 3: mU26 is transcribed in many tissues of mouse

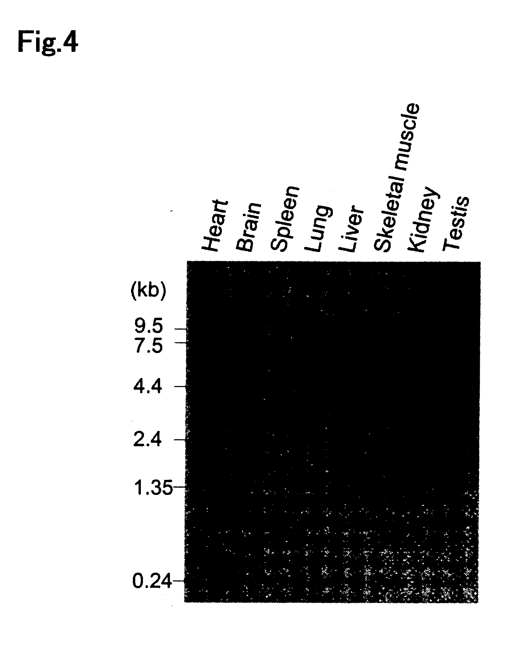

AAS synthetase (AASS) that converts lysine into AAS is transcribed in many organ tissues (Papes, F. et al. (1999) Biochem. J. 344, 555-563; and Sacksteder, K. A. et al. (2000) Am. J. Hum. Genet. 66, 1736-1743; refer to Figure 1). It was examined whether or not the gene expression of mU26 also exhibits a wide tissue distribution. Northern blotting analysis was carried out using poly(A)⁺RNA extracted from various tissues (wherein a block sheet was purchased from Clontech). As a result, it was found that the mU26 gene was expressed in all the examined tissues (Figure 4). The cDNA probe of mU26 labeled with the radioisotope ³²P was hybridized with 3 types of transcribed products with different lengths in all the tissues. Since mRNA comprising a large deletion in the translation region thereof had not been detected in the RT-PCR experiment (data is not shown), it was considered that these 3 types of transcribed products are splice variants in the 3'-non-translation region. The overall expression pattern of mU26 was similar to that of AASS, and their mRNA was expressed at a high level in the heart, liver, and kidney.

### Example 4: Expression of mU26 is enhanced by inception of high lysine feedstuff

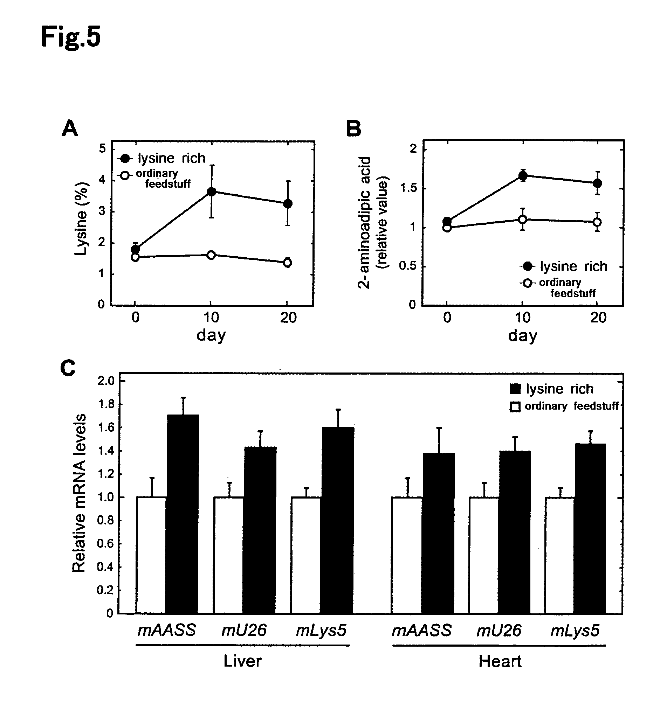

Whether or not mU26 is associated with lysine metabolism in mammals was examined using mice. Whether or not mU26 mRNA is increased by inception of feedstuff containing a large amount of lysine was analyzed. Feedstuff containing free lysine at an amount 2,000 times larger than that contained in ordinary feedstuff (obtained by adding 40 mg of L-lysine-hydrochloride to 1 g of ordinary feedstuff) was given to mice. Ten days after inception of such high lysine feedstuff, the amounts of lysine and AAA in blood were significantly increased, and such high level was maintained even on the 20^{th} day (Figures 5A, B). The amounts of lysine and AAA in blood were measured as follows. The peripheral blood (15 µl) was collected from the tail of a mouse, and trichloroacetic acid (final concentration: 4%) was added thereto. The mixture was centrifuged (18,000 x g), and the obtained supernatant was subjected to an amino acid analyzer (L-8500A; Hitachi), so as to quantify ordinary amino acids including lysine. For quantification of AAA, the supernatant obtained after centrifugation was hydrolyzed in the presence of 5 M hydrochloric acid (110°C, 2 hours), and it was then exsiccated. The resultant was dissolved in diluted hydrochloric acid, and it was then subjected to an amino acid analyzer again. Saccharopine and AAS contained in the blood were not detected with the amino acid analyzer using a ninhydrin reaction, probably because the amounts were extremely small. In addition, since AAS has extremely high reactivity (unstable) under conditions in living bodies (Basso, L.V. et al. (1962) J. Biol. Chem. 237, 2239-2245), it is considered that detection of AAS in blood would be most difficult.

The mRNA level of mU26 in the liver and heart (a relative value with respect to the mRNA level of mouse glyceraldehydes 3-phosphate dehydrogenase [mGAPDH]) was measured on the 20^{th} day after the mice had been fed with high lysine feedstuff. The mRNA level was quantified by the real-time PCR method (ABI 7000) using SYBR Green PCR Master Mix available from Applied Biosystems. The following primers were used for PCR.
mGAPDH

5’-TGCACCACCAACTGCTTAG-3’ (SEQ ID NO: 5)

5’-GGATGCAGGGATGATGTTC-3’ (SEQ ID NO: 6)
mAASS

5'-CGCAGACCAGCAAATTATTCAC-3' (SEQ ID NO: 7)

5’-ATTCCGTAGCCTCGATTGGA-3’ (SEQ ID NO: 8)
mU26

5’-CTGGTGAGAGCGGCTGTACA-3’ (SEQ ID NO: 9)

5'-GGACAGGTCGACAGCCTTCA-3' (SEQ ID NO: 10)
mLys5

5'-TGATGAATGGACTCAGCTGGAT-3' (SEQ ID NO: 11)

5’-CCTGACCAATGTCCATGTTTAATG-3’ (SEQ ID NO: 12)

As a result, it was found that the mU26 mRNA level of mice fed with high lysine feedstuff was 1.6 times (in the liver) and 1.4 times (in the heart) higher than that of mice fed with ordinary feedstuff (Figure 5C). As a control, the mRNA level of mAASS and that of mLys5 (mouse homologues of yeast Lys5; see Fig.1) were measured at the same time. As a result, it was found that both the mRNA levels were increased in the liver and heart of the mice fed with high lysine feedstuff. These experimental results show that a feedback system exists in the lysine metabolic system and that mU26 in addition to mAASS and mLYS5 is associated with lysine decomposition.

### Example 5: The level of aminoadipic acid in blood is decreased in PQQ-deficient mice

In order to clarify the role of PQQ influenced on AASDH activity, feedstuff containing no PQQ was given to mice, and such influence was examined. Chemically defined feedstuff (to which no PQQ was added or 100 ng PQQ·2Na was added per gram of feedstuff) was produced in accordance with the publication (Killgore, J. et al. (1989) Science 245, 850-852; Steinberg, F. M. et al. (1994) J. Nutr. 124, 744-753). The thus produced feedstuff was given to virgin female mice (ICR, 4-week-old) for 20 days. The levels of various amino acids and AAA in blood were measured by the above-described method. As a result, it was found that the amount of lysine is not changed by inception of PQQ-deficient feedstuff (Figure 6A), and that a well-balanced metabolism is conducted regarding lysine molecules themselves. As stated above, saccharopine and AAS were not detected, but it was found that the level of AAA in blood was significantly decreased in PQQ-deficient mice (Figures 6B, C). Such AAA reduction was always significantly observed, regardless of the type of amino acid (for example, glycine; Figure 6C) with which the amount of AAA was normalized. In particular, when normalization was carried out with the amount of lysine, AAA was reduced most clearly (Figure 6B). These results suggested that AASDH activity (that is, mU26) is reduced in PQQ-deficient mice, and that PQQ is required as an oxidization-reduction coenzyme for AASDH.

### INDUSTRIAL APPLICABILITY

According to the present invention, 2-aminoadipate 6-semialdehyde hydrogenase has been identified as an enzyme that utilizes pyrroloquinoline quinone (PQQ) as a coenzyme in mammals. As a result, an oxidization-reduction reaction, with which PQQ is associated as a *coenzyme in vivo,* has been specified, and the biochemical role of PQQ *in vivo* has been clarified. According to the present invention, a novel vitamin preparation can be provided by using PQQ.

## Claims

1. A method of using pyrroloquinoline quinone as a coenzyme for 2-aminoadipate 6-semialdehyde dehydrogenase.

2. A method for converting 2-aminoadipate 6-semialdehyde into 2-aminoadipic acid, which comprises allowing 2-aminoadipate 6-semialdehyde dehydrogenase to act on 2-aminoadipate 6-semialdehyde in the presence of pyrroloquinoline quinone.

3. A vitamin preparation which comprises pyrroloquinoline quinone as a coenzyme for 2-aminoadipate 6-semialdehyde dehydrogenase.
